# EUROPEAN PATENT APPLICATION

(11) **EP 4 574 107 A1**
(43) Date of publication of application: **25.06.2025**
(21) Application number: 23852620.6
(22) Date of filing: 10.08.2023
(51) Int. Cl.: A61F 9/007, G02C 7/00

(54) **CONTACT LENS-SHAPED INTRAOCULAR LIGHTING DEVICE**

(30) Priority: 10.08.2022 JP 2022128395
(71) Applicant: University of Tsukuba, Ibaraki 305-8577 (JP)
(72) Inventor: MORIKAWA, Shohei, Tsukuba-shi, Ibaraki 305-8577 (JP)
(74) Representative: Sonnenberg Harrison Partnerschaft mbB
(86) International application number: PCT/JP2023/029230
(87) International publication number: WO 2024/034658

(57) **Abstract**

A contact lens-shaped intraocular lighting device including: a contact lens including a first region having a dome shape transmitting light and provided in a center portion, a second region having a ring shape extending radially outward from an outer peripheral portion of the first region, and a third region having a ring shape extending radially outward from an outer peripheral portion of the second region and provided in contact with a sclera; and an irradiation unit provided in the second region of the contact lens and irradiating an eye with light by adjusting refraction or diffusion of light supplied from a light source.

## Description

### TECHNICAL FIELD

The present invention relates to a contact lens-shaped intraocular lighting device. The present application claims priority based on Japanese Patent Application No. 2022-128395 filed in Japan on August 10, 2022, the contents of which are incorporated herein by reference.

### BACKGROUND ART

Major problems obstructing social life of human include visual impairment. Known causes of visual impairment include eye diseases such as cataract, retinal detachment, age-related macular degeneration, and diabetic retinopathy, and these eye diseases are also causes of blindness. It is considered desirable to avoid progression of eye diseases by early diagnosis. For example, cataract is diagnosed by examining the transparency of a crystalline lens using a slit lamp microscope and can be treated by surgery. Retinal detachment is diagnosed by funduscopy and treated with vitreous surgery or scleral buckling procedure. Age-related macular degeneration is diagnosed by funduscopy and treatments therefore include photodynamic therapy. Diabetic retinopathy is diagnosed by a retinal photograph, and can be treated by laser photocoagulation, vitreous surgery, or the like. Diabetic retinopathy often has no early warning symptoms, but defect abnormalities such as capillary aneurysms, retinal hemorrhages, beading of retinal veins, and other micro-defect abnormalities appear in retinal photographs.

When an intraocular surgery such as vitreous surgery is performed, use of an intraocular lighting device is common. In this case, a port is formed at a position of about 3.5 mm to 4.0 mm from a corneoscleral limbus portion in the sclera, and the intraocular lighting device is inserted into the eye through the port (e.g., Patent Document 1).

Known photodynamic therapies known as treatment of macular degeneration include a treatment using a smart remotely controlled contact lens including a drug reservoir storing a photosensitive agent, a photodetector, and a micro-LED or -OLED (e.g., Patent Document 2). In the treatment using the smart remotely controlled contact lens, first, by a signal from the photodetector, the photosensitive agent stored in the drug reservoir is released through a system and transmitted to a retinal defect. Here, as the photosensitive agent, a material generating active oxygen in response to light is used. When the photosensitive agent is transmitted to the retinal defect, active oxygen is generated using light of the micro-LED or -OLED, and the generated active oxygen is used for treatment of macular degeneration.

Known means for taking a retinal photograph include eye diagnosis using an irradiation type contact lens assembly (e.g., Patent Document 3). A contact lens assembly includes: a contact lens including a proximal curved surface arranged on a side close to an eye and a distal flat surface that is a surface opposite to the proximal curved surface; a light source having an annular shape in plan view, the light source being provided outside a position away from the proximal curved surface more than the distal flat surface of the contact lens and coupled to an external light source through an optical fiber; a tubular reflector provided to surround the light source; and a camera provided on a distal curved surface of the contact lens. In this contact lens assembly, a light source provided outside the contact lens irradiates the proximal curved surface side with light to take a retinal photograph.

### Citation List

### Patent Document

Patent Document 1: JP 2019-511273 A
Patent Document 2: JP 2021-508860 A
Patent Document 3: JP 2022-508709 A

### SUMMARY OF INVENTION

### Technical Problem

In a case of using an invasive intraocular lighting device as in Patent Document 1, since the intraocular lighting device is inserted into the eye via the port, there is an infection risk due to a trace amount of bacteria and viruses remaining in the intraocular lighting device even if sterilization treatment is appropriately performed. In the case of using the intraocular lighting device described in Patent Document 1, it is necessary to operate the intraocular lighting device with one hand and perform intraocular treatment with the other hand, and therefore the treatment is difficult to perform.

The treatment method as in Patent Document 2 is effective without the need for inserting a sensitive agent by surgery, but is a specific treatment method in which active oxygen generated by using light of an LED is used for treatment of macular degeneration, and the application thereof is limited. The contact lens assembly as in Patent Document 3 is effective for taking a retinal photograph, but since it has a special shape and structure, use for internal eye surgery is not assumed and is not suitable. Specifically, the light source and the reflector provided outside the contact lens cannot follow the movement of the eyes, and may damage an eyelid and the like. Therefore, it is not suitable for long-term use such as intraocular surgery. Since the light source is provided outside the contact lens, it is difficult to illuminate the entire retina.

The present invention has been made in view of the above circumstances, and it is an object of the present invention to provide a contact lens-shaped intraocular lighting device suitable for bimanual intraocular surgery and having high versatility while reducing an infection risk as much as possible.

### Solution to Problem

(1) A contact lens-shaped intraocular lighting device according to one aspect of the present invention includes: a contact lens including a first region having a dome shape transmitting light and provided in a center portion, a second region having a ring shape extending radially outward from an outer peripheral portion of the first region, and a third region having a ring shape extending radially outward from an outer peripheral portion of the second region and provided in contact with a sclera; and an irradiation unit provided in the second region of the contact lens and irradiating an eye with light by adjusting refraction or diffusion of light supplied from a light source.
(2) In the contact lens-shaped intraocular lighting device of (1), the irradiation unit may include a holographic lens refracting and guiding, into an eye, light supplied from the light source, and a holographic lens drive circuit electrically connected with the holographic lens and changing an orientation of the holographic lens.
(3) In the contact lens-shaped intraocular lighting device of (1) or (2), the irradiation unit may include a plurality of holographic lens units each including the holographic lens and the holographic lens drive circuit, and a plurality of the holographic lens units may be provided side by side on an identical circumference of the contact lens in plan view of the contact lens.
(4) In the contact lens-shaped intraocular lighting device of any of (1) to (3), a plurality of the holographic lens units may be arranged rotationally symmetrically about a circle center of the contact lens in plan view of the contact lens.
(5) The contact lens-shaped intraocular lighting device of any of (1) to (4) may further include a light source drive circuit provided in the second region and driving the light source.
(6) In the contact lens-shaped intraocular lighting device of any of (1) to (5), the irradiation unit may include a light guide plate guiding, to a diffusion sheet, light supplied from the light source, and a diffusion sheet layered on the light guide plate, and diffusing and guiding, into an eye, light from the light guide plate.
(7) In the contact lens-shaped intraocular lighting device of (1), the irradiation unit may include a plurality of optical layered units each including the light guide plate and the diffusion sheet, and the plurality of optical layered units may be provided side by side on an identical circumference of the contact lens in plan view of the contact lens.
(8) In the contact lens-shaped intraocular lighting device of (7), a plurality of the optical layered units may be arranged rotationally symmetrically about a circle center of the contact lens in plan view of the contact lens.
(9) The contact lens-shaped intraocular lighting device of any of (1) to (8) may further include an antireflection film provided on a surface on an opposite side to a wearing surface of the contact lens, in which the antireflection film may be arranged to cover the irradiation unit in plan view of the contact lens.
(10) In the contact lens-shaped intraocular lighting device of any of (1) to (9), a thickness of the first region may be 0.15 mm or more and less than 0.25 mm.
(11) In the contact lens-shaped intraocular lighting device of any of (1) to (10), the contact lens may be a scleral lens having a diameter of 13 mm or more.
(12) In the contact lens-shaped intraocular lighting device of any of (1) to (11), the light source may be provided in the second region, and electric power may be supplied to the light source from an outside of the contact lens-shaped intraocular lighting device.
(13) In the contact lens-shaped intraocular lighting device of any of (1) to (12), an outer surface on an opposite side to a wearing surface of the contact lens may have a spherical surface corresponding to the first region, a first ring surface corresponding to the second region, the first ring surface being a continuous surface with the spherical surface, and a second ring surface corresponding to the third region.
(13) A contact lens-shaped intraocular lighting device according to one aspect of the present invention includes: a contact lens including a third region having a ring shape provided in contact with a sclera, and a second region having a ring shape extending radially inward from an inner peripheral portion of the third region; and an irradiation unit provided in the second region of the contact lens and irradiating an eye with light by adjusting refraction or diffusion of light supplied from a light source. The contact lens-shaped intraocular lighting device according to the present aspect may have at least one of the configurations (1) to (8) or (10) to (12).

### Advantageous Effects of Invention

According to the present invention, it is possible to provide a contact lens-shaped intraocular lighting device suitable for bimanual intraocular surgery and having high versatility while reducing an infection risk as much as possible.

### BRIEF DESCRIPTION OF DRAWINGS

FIG. 1 is a cross-sectional view illustrating an example of a configuration of a contact lens-shaped intraocular lighting device according to one embodiment of the present invention.
FIG. 2 is a plan view of the contact lens-shaped intraocular lighting device of FIG. 1 as viewed from an operator side (outer peripheral surface side).
FIG. 3 is a view illustrating the contact lens-shaped intraocular lighting device of FIG. 1 being attached to an eye of a patient.
FIG. 4 is a cross-sectional view illustrating a modification of the contact lens-shaped intraocular lighting device of FIG. 1.
FIG. 5 is a cross-sectional view illustrating an example of a configuration of a contact lens-shaped intraocular lighting device according to another embodiment of the present invention.
FIG. 6 is a plan view of the contact lens-shaped intraocular lighting device of FIG. 5 as viewed from an operator side (outer peripheral surface side).
FIG. 7 is a plan view illustrating a modification of the contact lens-shaped intraocular lighting device of FIG. 6.
FIG. 8 is a cross-sectional view illustrating a modification of the contact lens-shaped intraocular lighting device of FIG. 5.

### DESCRIPTION OF EMBODIMENTS

Hereinafter, an example of an embodiment of the present invention will be described in detail with reference to the drawings. Note that in the drawings used in the following description, a characteristic part may be enlarged for convenience and thus makes the features of the present invention easy to understand. For this reason, the dimensional ratio and the like of each component may be different from an actual one.

### [Contact Lens-Shaped Intraocular Lighting Device]

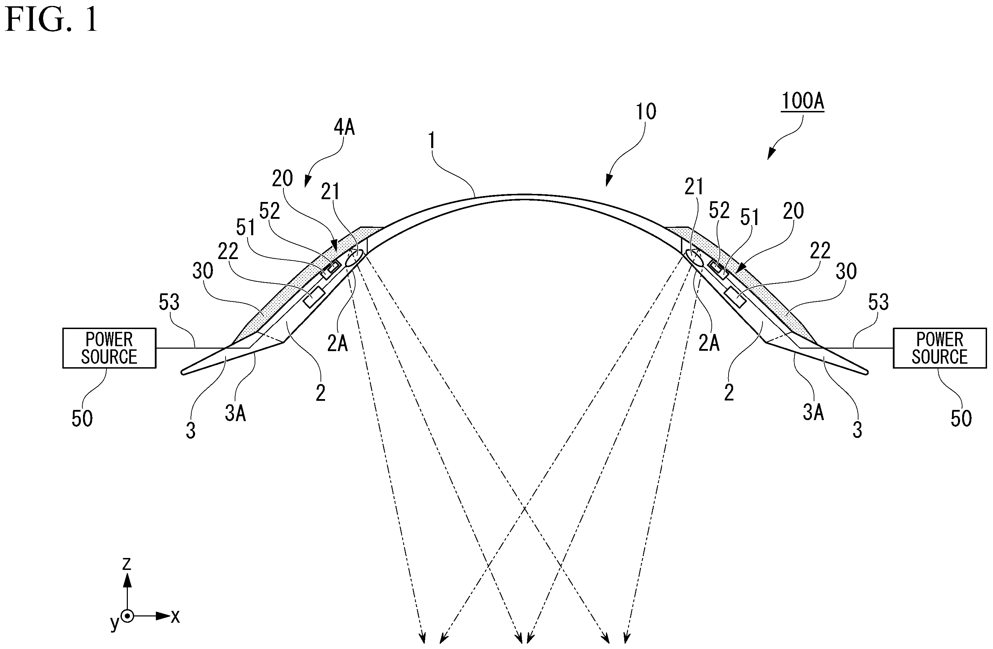

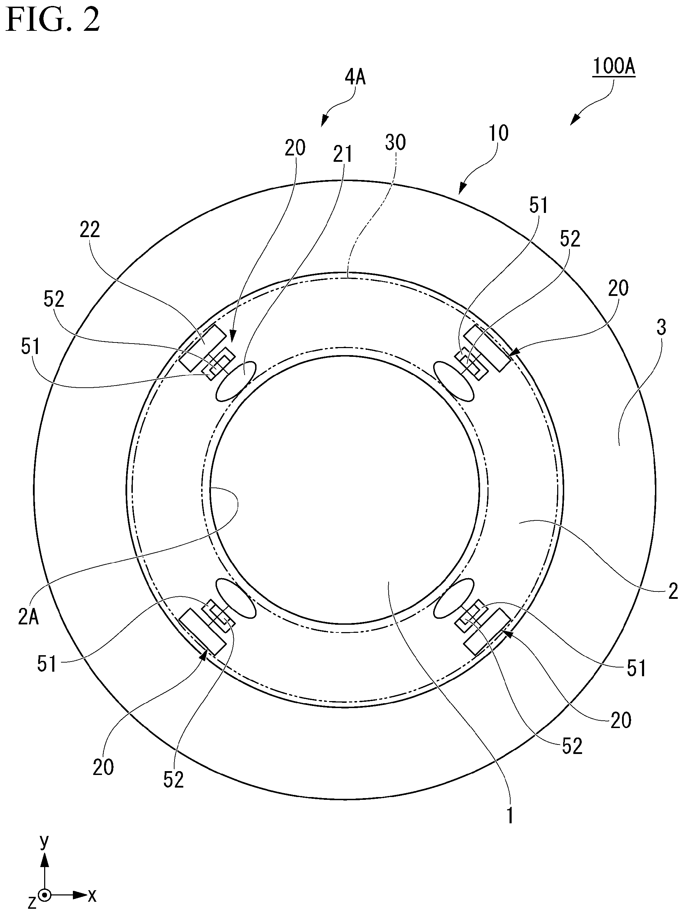

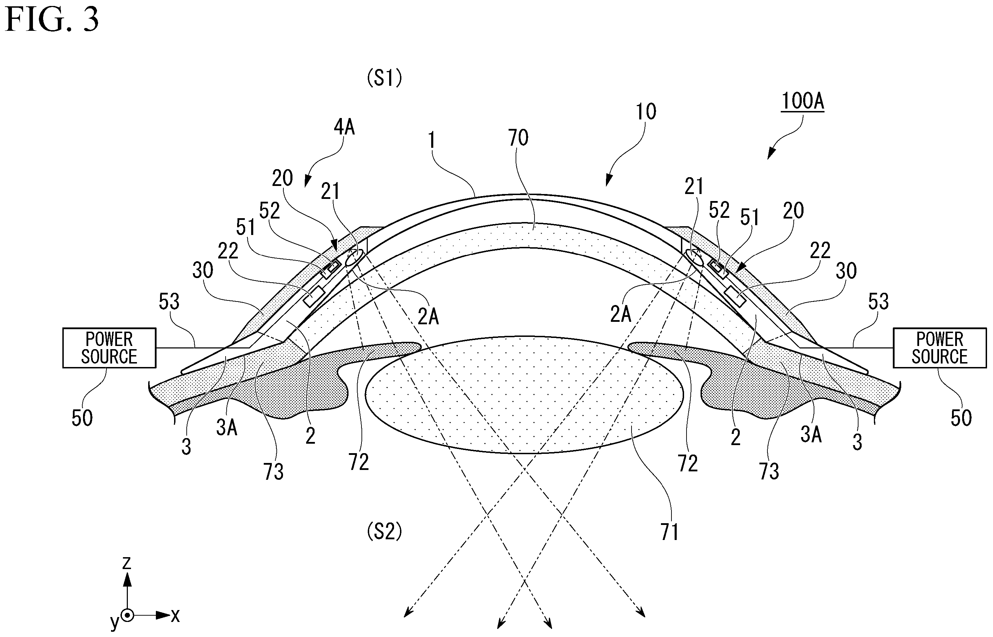

FIG. 1 is a cross-sectional view illustrating an example of the configuration of a contact lens-shaped intraocular lighting device according to one embodiment of the present invention, FIG. 2 is a plan view of the contact lens-shaped intraocular lighting device of FIG. 1 as viewed from the operator side (outer peripheral surface side), and FIG. 3 is a view illustrating the contact lens-shaped intraocular lighting device of FIG. 1 being attached to an eye of a patient. In FIGS. 1 and 3, for convenience of description, light with which the eye is irradiated is indicated by a two-dot chain line. For convenience of description, FIG. 3 illustrates a cornea 70, a crystalline lens 71, an iris 72, and a sclera 73.

A contact lens-shaped intraocular lighting device 100A illustrated in FIGS. 1 to 3 includes: a contact lens 10 including a first region 1 having a dome shape transmitting light and provided in a center portion, a second region 2 having a ring shape extending radially outward from an outer peripheral portion of the first region 1, and a third region 3 having a ring shape extending radially outward from an outer peripheral portion of the second region 2 and provided in contact with a sclera 73; and an irradiation unit 4A provided in the second region 2 of the contact lens 10 and irradiating an eye with light by adjusting refraction or diffusion of light supplied from a light source. In the present embodiment, the center portion means that it is positioned in the vicinity of the center in plan view and is not positioned at the end. That is, the center portion is surrounded by other regions. In the present embodiment, the outer peripheral portion means a region separated radially outward from the center in plan view, the outer peripheral portion of the first region 1 is a part closest to the second region 2 in the first region 1, and the outer peripheral portion of the second region 2 is a part closest to the third region in the second region 2.

### <Contact Lens>

The contact lens 10 is a scleral lens including the first region 1, the second region 2, and the third region 3 in order from the radially inside. In the contact lens 10, the first region 1, the second region 2, and the third region 3 may be called an optic zone, a Z-zone, and a landing zone, respectively. In the contact lens 10, the first region 1 is a region for the operator to observe the inside of the eye with a microscope, for example. The second region 2 is a region where the irradiation unit 4A is formed, and is positioned between the first region 1 and the third region 3. The third region 3 is a region for attaching the contact lens 10 to the sclera 73 of the patient. Of the third region 3, a wearing surface attached to the sclera 73 is indicated by reference sign 3A. Of the surfaces of the contact lens 10, the surface opposite to the wearing surface 3A is called an outer surface. The outer surface of the contact lens 10 includes, for example, a spherical surface corresponding to the first region 1, a first ring surface corresponding to the second region 2, the first ring surface being a continuous surface with the spherical surface, and a second ring surface corresponding to the third region 3, the second ring surface being a continuous surface with the first ring surface. In FIG. 1, the boundary between the first region 1 and the second region 2 is indicated by a solid line, and the boundary between the second region and the third region 3 is indicated by a two-dot chain line. The spherical surface is a surface on an outer (S1) side of the first region 1, the first ring surface is a surface on the outer (S1) side of the second region 2, and the second ring surface is a surface on the outer (S1) side of the third region.

A diameter (DIA) of the contact lens 10 is, for example, 13 mm or more, and may be 15 mm or more or 16 mm or more. Here, the diameter satisfies both the horizontal diameter and the vertical diameter.

In the contact lens 10, the first region 1 is, for example, a region having a diameter of 3.0 to 6.0 mm including the center in plan view of the contact lens 10. The thickness of the first region 1 may vary depending on the position, but may be, for example, 0.1 mm or more and less than 0.5 mm, 0.15 mm or more and less than 0.25 mm, or 0.15 mm or more and less than 0.20 mm. Since the contact lens-shaped intraocular lighting device 100A according to the present embodiment is used in surgery, it can be used even with a thin structure in which the thickness of the first region 1 is less than 0.25 mm. When the thickness of the first region 1 is as small as 0.25 mm or the like, it is preferable from the viewpoint of preventing inhibition of the observation system in internal eye surgery.

The second region 2 is a region positioned in the outer peripheral direction relative to the first region 1, and has a radial size (width) of, for example, 2.0 to 5.0 mm. The second region 2 is a region continuous with the first region 1 and the third region 3, and has, for example, an inclined structure having a gradient steeper than that of the third region 3. The outer peripheral portion of the second region 2 is, for example, at a position 10 to 13 mm from the center in plan view of the contact lens 10. The thickness of the second region 2 increases as separated radially from the circle center, for example. The thickness of the second region 2 is larger than the thickness of the first region 1, and is 0.3 mm or more and less than 2.0 mm, and preferably 0.3 mm or more and less than 0.75 mm. Since the irradiation unit 4A is formed in the second region 2, the thickness is sufficiently large to secure the strength, and the thickness is sufficiently small to suppress an influence on other surgical instruments and observation systems. Hereinafter, the center in plan view of the contact lens 10 may be simply called a circle center.

As described above, the third region 3 is provided in contact with the sclera 73. The radial width of the third region 3 is, for example, 1 to 2 mm. The third region 3 is provided in contact with the sclera 73 on the wearing surface 3A.

The contact lens 10 is made of a material such as glass, methacrylic resin, or acrylic resin, for example.

The first region 1, the second region 2, and the third region 3 constituting the contact lens 10 are provided integrally, for example, and are formed of an identical material. However, the present invention is not limited to this, and the first region 1, the second region 2, and the third region 3 may be shaped of different materials, and the contact lens 10 may be formed by bonding them.

### <Antireflection Film>

The contact lens-shaped intraocular lighting device 100A further includes an antireflection film 30 on a surface opposite to the wearing surface 3A of the contact lens 10. The antireflection film 30 is arranged to cover the irradiation unit 4A in plan view of the contact lens 10. The antireflection film 30 has, for example, an annular shape in plan view. The antireflection film 30 is provided overlapping, for example, at least the second region 2, and may be provided overlapping the second region 2 and the third region 3. The cross-sectional view shape of the antireflection film 30 is, for example, a shape along the surface opposite to the wearing surface 3A of the contact lens 10. The antireflection film 30 is typically provided in direct contact with the second region 2, but is not limited to this, and may be provided via another film.

The antireflection film 30 is a film having an antireflection structure, and is, for example, a black filter, an alternately layered structure of a layer of a high refractive index material and a layer of a low refractive index material, a film having a moth eye structure, or the like. The antireflection film 30 takes a role of absorbing light from the outside (S1) of the contact lens-shaped intraocular lighting device 100A such as a light source included in the surgical microscope, for example. The antireflection film 30 takes a role of absorbing light that enters the eye from a light source 52 through the first region 1 and is reflected in an eye (S2), for example. Providing the antireflection film 30 allows the operator to easily observe the inside of the eye during the intraocular surgery.

### <Irradiation Unit>

The irradiation unit 4A is provided, for example, in the second region 2 of the contact lens 10. The irradiation unit 4A takes a role of illuminating the inside of the eye of the patient. For example, the irradiation unit 4A illuminates the inside of the eye of the patient and thus the center illuminance at an ocular fundus is 10000 to 100000 lx. The illuminance described above is in compliance with JIS Z9110:2010.

The irradiation unit 4A includes at least one holographic lens 21 refracting and guiding, into the eye, light supplied from the light source 52, and at least one holographic lens drive circuit 22 electrically connected to the holographic lens 21 and changing the orientation of the holographic lens 21. Hereinafter, the holographic lens 21 and the holographic lens drive circuit 22 are collectively called a holographic lens unit 20. The irradiation unit 4A includes at least one light source 52 and at least one holographic lens unit 20, and from the viewpoint of increasing the illuminance, preferably includes a plurality of holographic lens units 20 and light sources 52, and more preferably includes three or more of them. For example, the holographic lens units 20 are provided in a one-to-one connection corresponding to the light sources 52. Specifically, a radial inside of the contact lens 10 with respect to the light source 52 is provided with the holographic lens 21 described later, and thus the eye can be irradiated with light from the light source. The number of the holographic lens units 20 and the number of the light sources 52 are, for example, the same.

### (Light Source Drive Circuit)

The irradiation unit 4A further includes, for example, a light source drive circuit 51 driving the light source 52. The light source drive circuit 51 includes, for example, the light source 52 and a circuit driving the light source 52. The light source 52 is, for example, a substrate layered type LED, a halogen lamp, a xenon lamp, or the like.

The light source drive circuit 51 is provided, for example, in the second region 2 of the contact lens 10. The light source drive circuit 51 and the light source 52 are supplied with electric power from the outside of the contact lens-shaped intraocular lighting device 100A, for example. The connection between the light source drive circuit 51 and a power source 50 may be a wireless connection or a wired connection. When the connection between the light source drive circuit 51 and the power source 50 is a wireless connection, the power source 50 is, for example, a wireless drive power source provided outside the contact lens 10, and wirelessly supplies electric power to the light source drive circuit 51. When the connection between the light source drive circuit 51 and the power source 50 is a wired connection, the second region 2 and the third region 3 of the contact lens 10 are provided with an electric power supply circuit 53.

### (Holographic Lens Unit)

The plurality of holographic lens units 20 are preferably arranged on an identical circumference at an equal distance from the center in plan view of the contact lens 10 in plan view of the contact lens 10. Since the holographic lens units 20 are provided side by side along the circumference, it is possible to suppress variations in illuminance in the eye in the radial direction of the contact lens 10. The plurality of holographic lens units 20 are preferably arranged rotationally symmetrically about a circle center of the contact lens 10 in plan view of the contact lens 10. Since the plurality of holographic lens units 20 are arranged rotationally symmetrically about the circle center of the contact lens 10, it is possible to suppress variations in illuminance in the circumferential direction of the contact lens 10.

FIG. 2 illustrates an example in which the four holographic lens units 20 are arranged rotationally symmetrically by 90° on the identical circumference at an equal distance from the center in plan view of the contact lens 10, but the number of the holographic lens units 20 is discretionary, and the arrangement thereof can also be discretionary. The contact lens-shaped intraocular lighting device 100A may include, for example, three holographic lens units 20 and may be arranged rotationally symmetrically at 120° about the circle center of the contact lens 10, or may include six holographic lens units 20 and may be arranged rotationally symmetrically at 60° about the center of a circle in plan view of the contact lens 10.

Each of the holographic lens units 20 includes, for example, the holographic lens 21 refracting and guiding, into the eye, light supplied from the light source 52, and the holographic lens drive circuit 22 electrically connected to the holographic lens 21 and changing the orientation of the holographic lens 21. The holographic lens 21 is supported by the contact lens 10 with a degree of freedom of movement. For example, the holographic lens 21 is supported, and thus the angle of an incident surface of the holographic lens 21 on which light from the light source 52 is incident can be changed with respect to the incident light.

The holographic lens 21 and the light source 52 are preferably arranged on an inner peripheral surface side (side close to the eye) of the contact lens 10. For example, the holographic lens 21 is provided between the center position in the thickness direction of the second region 2 and an inner peripheral surface 2A, and is preferably provided on the inner peripheral surface 2A of the second region 2. Such an arrangement of the holographic lens 21 makes it possible to suppress an excessive divergence angle of refracted light in the holographic lens 21 of the holographic lens unit 20, and makes it easy to adjust the irradiation position in the eye.

The holographic lens 21 moves relative to the contact lens 10 by a signal from the holographic lens drive circuit 22, and the traveling direction and the divergence angle of the light are changed along with the movement of the holographic lens 21.

### [Manufacturing Method for Contact Lens-Shaped Intraocular Lighting Device]

The contact lens-shaped intraocular lighting device according to the present embodiment is manufactured, for example, by a method including a step for producing a contact lens (lens producing) and a step for forming an irradiation unit on the contact lens (irradiation unit forming).

### <Step for Producing Lens>

First, a member constituting the contact lens 10 is produced. For example, two members having an identical shape in plan view that overlap in the thickness direction to form the contact lens 10 are formed. The member constituting the contact lens 10 is produced by, for example, means such as lace cutting, spin casting, and molding.

### <Step for Forming Irradiation Unit>

Next, the irradiation unit 4A is formed in a region to be the second region 2 of the contact lens 10. For example, the irradiation unit 4A is mounted on one surface of a member constituting the contact lens 10. The surface mounted with the irradiation unit 4A is an inner surface of the contact lens 10. That is, it is a surface on a side away from the operator side.

Next, for example, members constituting the contact lens 10 are layered, and thus the irradiation unit 4A is sandwiched between the members constituting the contact lens 10.

Next, the antireflection film 30 is formed on a surface opposite to the wearing surface 3A of the surface of the contact lens 10. The antireflection film 30 is formed using, for example, a transparent adhesive. Through the above process, the contact lens-shaped intraocular lighting device 100A is manufactured.

For example, as illustrated in FIG. 3, the contact lens-shaped intraocular lighting device 100A is easily worn to the eye of a patient by a method similar to a general wearing method for a vision correction contact lens. At this time, the third region 3 is placed in contact with the sclera 73, whereby the first region 1 and the second region 2 are arranged above the cornea. In this state, when the light source drive circuit 51 is given an instruction input from the outside, electric power is supplied from the power source 50 to the light source 52, and light is emitted from the light source 52, the eye is irradiated with light via the holographic lens 21. By giving the holographic lens drive circuit 22 an instruction input from the outside as necessary, the holographic lens drive circuit 22 can drive the holographic lens 21, and the irradiation position can be appropriately adjusted depending on the orientation of the holographic lens 21. By this, for example, a discretionary position in the eye such as the crystalline lens, the vitreous body, or the fundus is irradiated with light.

According to the contact lens-shaped intraocular lighting device 100A according to the present embodiment, since the irradiation unit 4A is provided in the second region 2 of the scleral lens, and irradiates the inside of the eye with light by adjusting refraction of light supplied from the light source 52, the operator can irradiate the inside of the eye with light without having the intraocular lighting device during surgery. Therefore, the contact lens-shaped intraocular lighting device 100A according to the present embodiment is suitable for bimanual intraocular treatment. Since it is not necessary to provide the sclera 73 or the like with a port for inserting the intraocular lighting device, the infection risk can be reduced. Furthermore, since it is possible to irradiate various sites in the eye only by wearing the contact lens-shaped intraocular lighting device 100A on the eye, it is possible to provide a highly versatile lighting device applicable to various surgeries.

Since the irradiation unit 4A is provided in the second region 2 of the contact lens 10, it is possible to perform intraocular lighting by a non-invasive means that does not require formation of a port, it is possible to follow eye movement, and it is less likely to damage an eyelid and the like. Therefore, the contact lens-shaped intraocular lighting device 100A is also suitable for long-term use such as intraocular surgery.

Furthermore, since in the contact lens 10 of the contact lens-shaped intraocular lighting device 100A, the third region 3 is provided in contact with the sclera 73 and the irradiation unit 4A is formed in the second region 2, the position of the contact lens-shaped intraocular lighting device 100 is hardly shifted. Specifically, the contact lens 10 is strongly supported between the sclera 73 and the eyelid in the third region 3, and the movement of the contact lens 10 itself is reduced. Even when eye movement occurs during surgery, the positional deviation can be reduced. Therefore, according to the contact lens-shaped intraocular lighting device 100, it is possible to reduce temporal variations in illuminance with respect to a specific position in the eye, and the visibility during surgery is stabilized, and therefore the observation system can be further stabilized.

### Modified Example

The present invention is not limited to the contact lens-shaped intraocular lighting device 100A according to the above embodiment, and can be appropriately modified within the scope of the gist described in the claims. For example, the present invention may be a contact lens-shaped intraocular lighting device not provided with the antireflection film 30. The irradiation unit 4A may be mounted in contact with the second region 2 of the contact lens 10.

FIGS. 1 to 3 illustrate an example in which the light source 52 is formed in the second region 2 of the contact lens 10, but the light source may be outside the contact lens 10. For example, the light source is arranged side by side with the contact lens 10 in an outer peripheral direction of the contact lens 10. When the light source is outside the contact lens 10, light having high directivity is emitted as the light source. The light from the light source passes through, for example, the third region 3, is emitted to the inside of the contact lens 10, is reflected inside the contact lens 10, enters the holographic lens 21, and irradiates the inside of the eye by refraction of the light at the holographic lens 21.

For example, FIGS. 1 to 3 illustrate an example in which the power source 50 is provided outside the contact lens 10 and is connected with the light source 52 in a wired or wireless manner, but the power source 50 may be provided in the contact lens 10. For example, the power source 50 may be provided in the second region 2. The number of power sources 50 may correspond to the number of light sources 52, or may be smaller than the number of light sources 52. When the number of power sources 50 is smaller than the number of light sources 52, a conductive wire continuous with the power source 50 may be provided along the shape of the second region 2 in the second region 2 of the contact lens 10 and connected with the plurality of light sources 52. In such a configuration, the plurality of light sources 52 are connected in parallel.

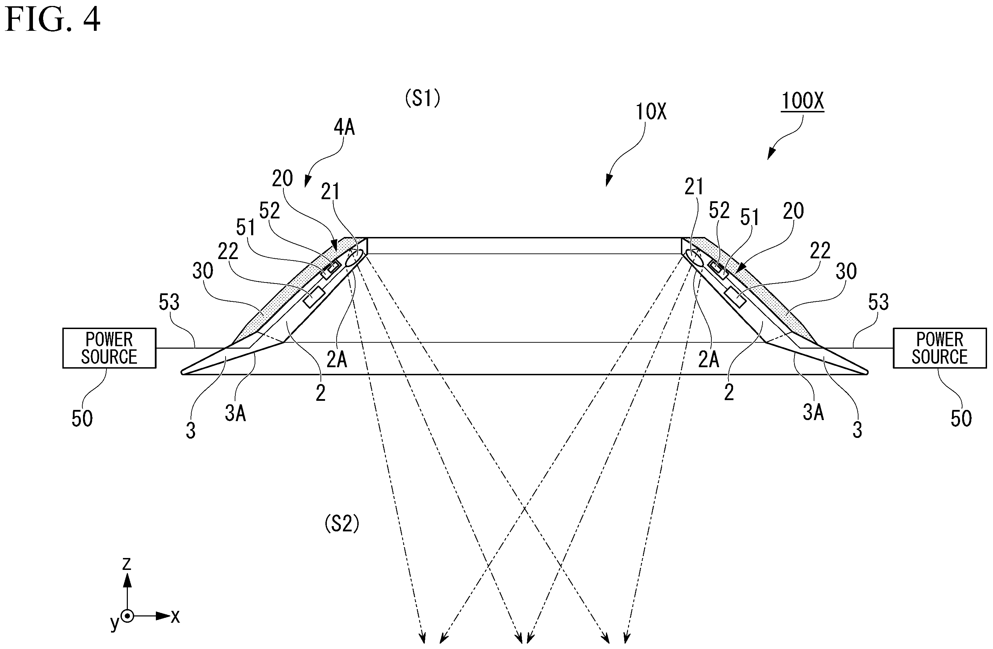

For example, the present invention may be a contact lens-shaped intraocular lighting device as illustrated in FIG. 4. FIG. 4 is a cross-sectional view illustrating a modification of the contact lens-shaped intraocular lighting device of FIG. 1. In the following drawings, similar components to those in FIGS. 1 to 3 are denoted by similar reference signs, and description thereof will be omitted. The following drawings omit illustration of the eye of the patient, and only illustrate a reference sign (S2) representing being in the eye.

A contact lens-shaped intraocular lighting device 100X illustrated in FIG. 4 includes a contact lens 10X including the third region 3 having a ring shape provided in contact with the sclera 73 and the second region 2 having a ring shape extending radially inward from an inner peripheral portion of the third region 3, and the irradiation unit 4A provided in the second region 2 of the contact lens 10X and irradiating an eye with light by adjusting refraction of light supplied from the light source 52. The contact lens 10X is different from the contact lens 10 in not including the first region 1. The contact lens 10X includes, for example, the second region 2 and the third region 3, and further includes the antireflection film 30 on the surface opposite to the wearing surface 3A.

The contact lens-shaped intraocular lighting device 100X is manufactured, for example, by a method for manufacturing the contact lens-shaped intraocular lighting device 100 and then further including a step for opening of forming an opening at the center in plan view of the contact lens 10.

The contact lens-shaped intraocular lighting device 100X also gives similar effects to those of the contact lens-shaped intraocular lighting device 100.

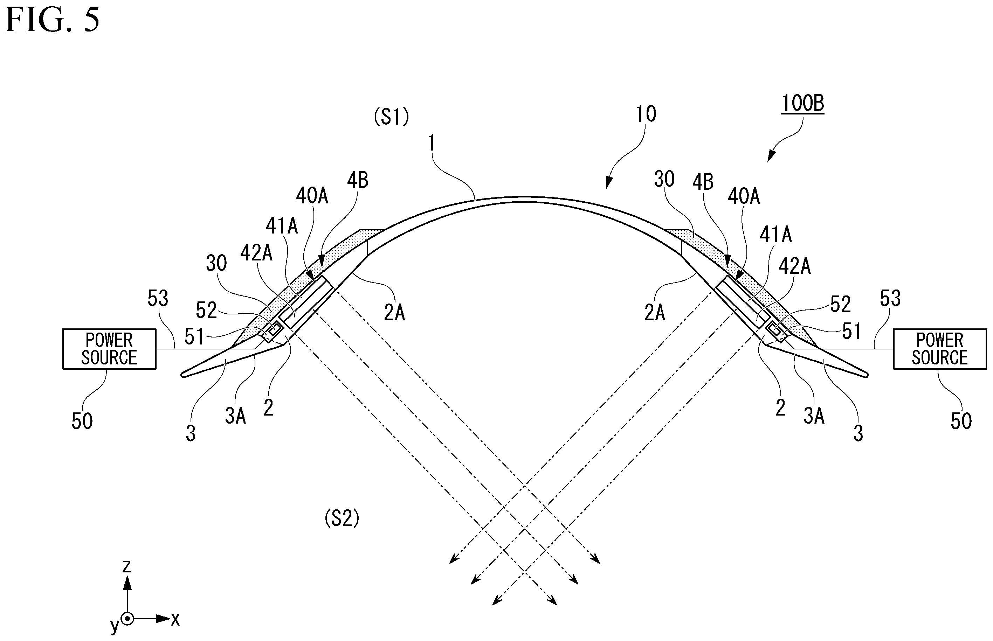

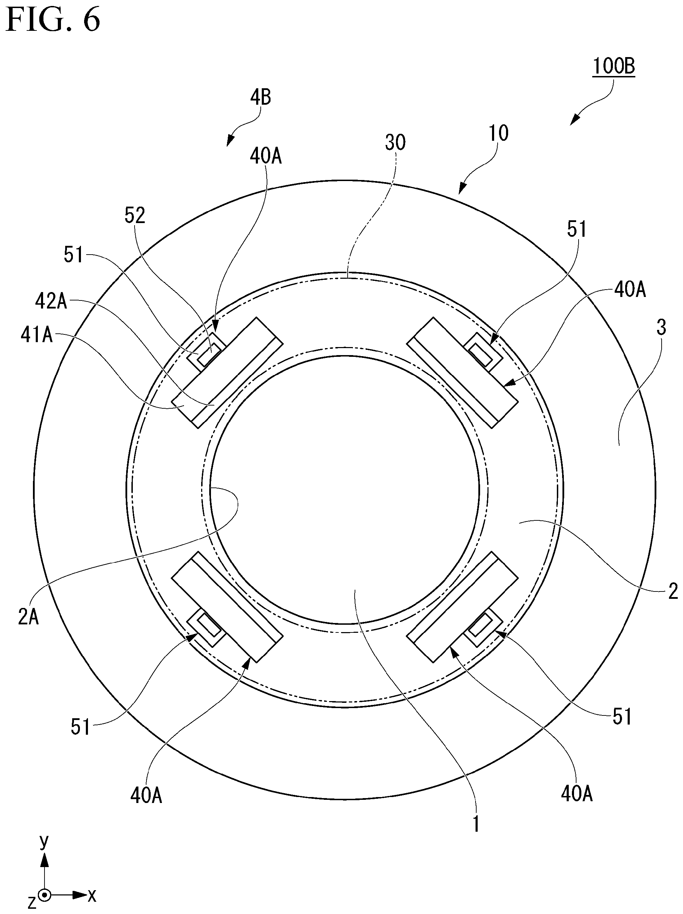

The present invention may be, for example, a contact lens-shaped intraocular lighting device 100B as illustrated in FIG. 5. FIG. 5 is a cross-sectional view illustrating an example of the configuration of the contact lens-shaped intraocular lighting device according to another embodiment of the present invention, and FIG. 6 is a plan view of the contact lens-shaped intraocular lighting device of FIG. 5 as viewed from the operator side (outer peripheral surface side). In FIG. 5, for convenience of description, light with which the eye is irradiated is indicated by a two-dot chain line.

The contact lens-shaped intraocular lighting device 100B illustrated in FIGS. 5 and 6 may include the contact lens 10, and at least one, preferably a plurality of, or more preferably three or more, irradiation units 4B provided in the second region 2 of the contact lens 10 and irradiating the inside of the eye with light by adjusting diffusion of the light supplied from the light source. For example, the irradiation unit 4B diffusely reflects the light supplied from the light source and irradiates the inside of the eye with light.

The irradiation unit 4B is provided, for example, in the second region 2 of the contact lens 10. The irradiation unit 4B takes a role of illuminating the inside of the eye of the patient. As an example, the irradiation unit 4B irradiates the inside of the eye of the patient and thus the center illuminance at an ocular fundus is 10000 to 100000 lx. The illuminance described above is in compliance with JIS Z9110:2010.

The irradiation unit 4B includes at least one light guide plate 41A guiding, to a diffusion sheet 42A, light supplied from the light source 52, and at least one diffusion sheet 42A layered on the light guide plate 41A, and diffusing and guiding, into an eye, light from the light guide plate 41A. Hereinafter, the light guide plate 41A and the diffusion sheet 42A are collectively called an optical layered unit 40A. The irradiation unit 4B includes at least one light source 52 and at least one optical layered unit 40A, and from the viewpoint of increasing the illuminance, preferably includes a plurality of the light sources 52 and optical layered units 40A. For example, the optical layered units 40A are provided in a one-to-one connection corresponding to the light sources 52. Specifically, an inner peripheral surface side (side close to the eye) of the contact lens 10 with respect to the light source 52 is provided with the optical layered unit 40A described later. The optical layered units 40A and the light sources 52 are the same in number, for example.

### (Light Source Drive Circuit)

The irradiation unit 4B further includes, for example, the light source drive circuit 51 driving the light source 52. The light source drive circuit 51 includes, for example, the light source 52 and a circuit driving the light source 52. The light source 52 included in the light source drive circuit 51 is arranged to irradiate the light guide plate 41A with light.

### (Optical Layered Unit)

The plurality of optical layered units 40A are preferably arranged on an identical circumference at an equal distance from the center of a circle in plan view of the contact lens 10 in plan view of the contact lens 10. Since the optical layered units 40A are provided side by side on the circumference, it is possible to suppress variations in illuminance in the eye in the radial direction of the contact lens 10. The plurality of optical layered units 40A are preferably arranged rotationally symmetrically about the circle center of the contact lens 10 in plan view of the contact lens 10. Since the plurality of optical layered units 40A are arranged rotationally symmetrically about the circle center of the contact lens 10, it is possible to suppress variations in illuminance in the circumferential direction of the contact lens.

FIG. 6 illustrates an example in which the four optical layered units 40A are arranged rotationally symmetrically by 90° on the identical circumference at an equal distance from the center in plan view of the contact lens 10, but the number of the optical layered units 40A is discretionary, and the arrangement thereof can also be discretionary. For example, the contact lens-shaped intraocular lighting device 100B may include three optical layered units and may be arranged rotationally symmetrically by 120° about the center of a circle in plan view of the contact lens 10, or may include six optical layered units and may be arranged rotationally symmetrically by 60° about the center of a circle in plan view of the contact lens 10.

Each of the optical layered units 40A includes, for example, the light guide plate 41A and the diffusion sheet 42A layered on an inner peripheral surface side of the contact lens 10 with respect to the light guide plate 41A. In the present embodiment, the light guide plate 41A and the diffusion sheet 42B are arranged substantially perpendicular to the thickness direction of the contact lens 10. The thickness of the diffusion sheet 42A is larger than the thickness of the light guide plate 41A, for example. For example, the light guide plate 41A emits, from a main surface of the light guide plate 41A, light incident from a side surface of the light guide plate 41A, and guides the light to the diffusion sheet 42B. The diffusion sheet 42B transmits and diffuses light incident from one main surface, for example, and emits diffused light from the other main surface.

The optical layered unit 40A and the light source 52 are preferably arranged on the inner peripheral surface side of the contact lens 10. For example, the end surface on the inner peripheral surface side of the optical layered unit 40 is provided between the center position in the thickness direction of the second region 2 and an inner peripheral surface 2A, and is preferably provided on the inner peripheral surface 2A of the second region 2. Such an arrangement of the light guide plate 41A and the diffusion sheet 42A can secure illuminance and intensity.

Similar to the contact lens-shaped intraocular lighting device 100A, the contact lens-shaped intraocular lighting device 100B is easily worn to the eye of a patient, and the third region 3 is placed in contact with the sclera 73. Then, when the light source drive circuit 51 is given an instruction input from the outside, electric power is supplied from the power source 50 to the light source 52, and light is emitted from the light source 52, the light enters the diffusion sheet 42A through the light guide plate 41A, and the diffused light generated in the diffusion sheet 42A is emitted into the eye. By this, for example, a discretionary position in the eye such as the crystalline lens, the vitreous body, or the fundus is irradiated with light.

According to the contact lens-shaped intraocular lighting device 100B according to the present embodiment, since the irradiation unit 4B is provided in the second region 2 of the scleral lens, and irradiates the inside of the eye with light by adjusting diffusion of light supplied from the light source 52, the operator can irradiate the inside of the eye with light without holding the intraocular lighting device during surgery. Therefore, the contact lens-shaped intraocular lighting device 100B according to the present embodiment is suitable for bimanual intraocular treatment. Since it is not necessary to provide the sclera 73 or the like with a port for inserting the intraocular lighting device, the infection risk can be reduced. Furthermore, since it is possible to irradiate various sites in the eye only by wearing the contact lens-shaped intraocular lighting device 100B on the eye, it is possible to provide a highly versatile lighting device applicable to various surgeries.

Since the irradiation unit 4B is provided in the second region 2 of the contact lens 10, similarly to the contact lens-shaped intraocular lighting device 100A, the irradiation unit 4B is non-invasive, can follow eye movement, and hardly damages an eyelid and the like. Therefore, the contact lens-shaped intraocular lighting device 100B is suitable for long-term use such as intraocular surgery.

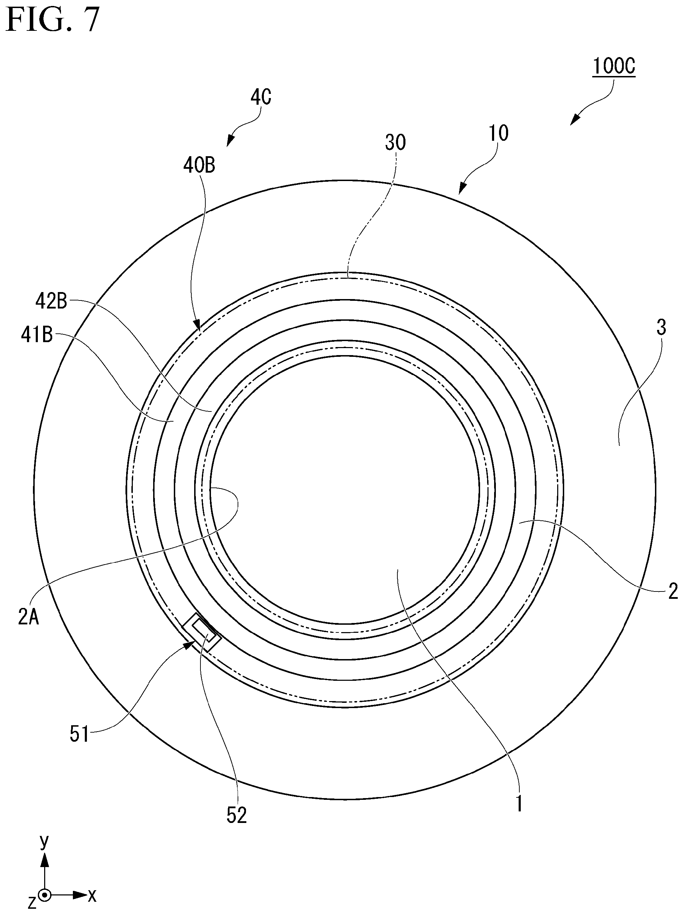

FIG. 7 is a plan view illustrating a modification of the contact lens-shaped intraocular lighting device of FIG. 6. A contact lens-shaped intraocular lighting device 100C illustrated in FIG. 7 includes the contact lens 10 and an irradiation unit 4C provided in the second region 2 and irradiating the inside of the eye with light by adjusting reflection of light supplied from the light source 52. The irradiation unit 4C includes, for example, a light guide plate 41B having an annular shape in plan view and a diffusion sheet 42B having an annular shape in plan view layered on an inner peripheral surface side (surface side close to the eye) with respect to the light guide plate 41B. The light guide plate 41B and the diffusion sheet 42B are collectively called an optical layered unit 40B. In the optical layered unit 40B, the thickness of the diffusion sheet 42B is larger than the thickness of the light guide plate 41A, for example. According to the contact lens-shaped intraocular lighting device 100C, it is possible to suppress variations in illuminance in the circumferential direction in the eye.

The contact lens-shaped intraocular lighting devices 100B and 100C are manufactured by a similar method to that of the contact lens-shaped intraocular lighting device 100A except forming the irradiation unit 4B or 4C in the step for forming irradiation unit.

In the contact lens-shaped intraocular lighting devices 100B and 100C, examples in which the number of the light sources 52, the number of the light guide plates 41A or 41B, and the number of the diffusion sheets 42A or 42B are the same, but they need not be the same. For example, more light sources 52 may be formed than the light guide plates 41A or 41B, and one light guide plate 41A or 41B may be irradiated with light from a plurality of light sources. This configuration can further increase the illuminance. More light guide plates 41A or 41B may be formed than the diffusion sheets 42A or 42B, and one diffusion sheet 42A or 42B and a plurality of light guide plates 41A or 41B may be layered.

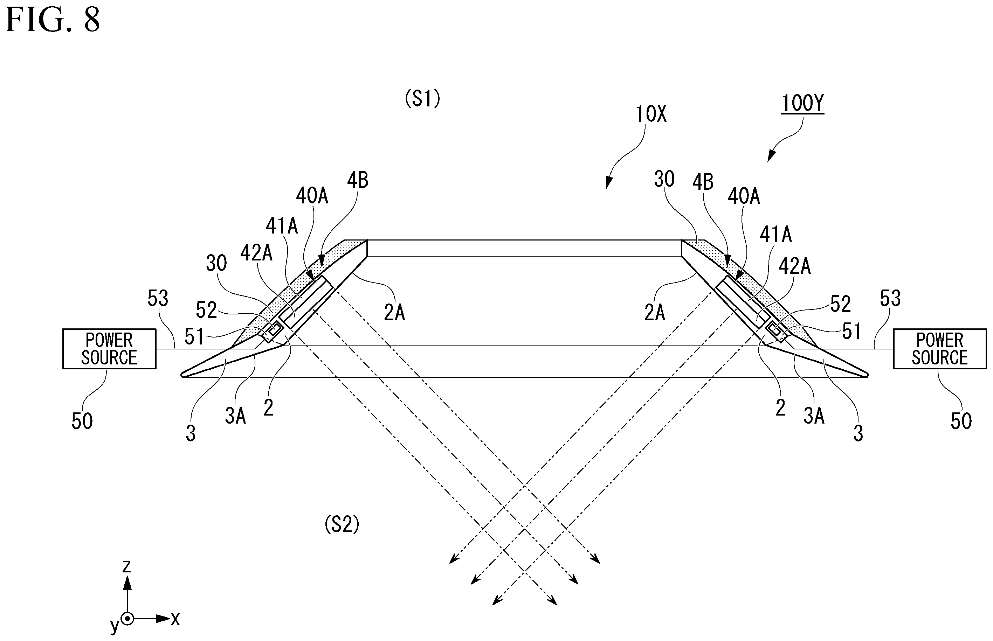

For example, the present invention may be a contact lens-shaped intraocular lighting device as illustrated in FIG. 8. FIG. 8 is a cross-sectional view illustrating a modification of the contact lens-shaped intraocular lighting device of FIG. 5. In the following drawings, similar components to those in FIGS. 1 to 3 are denoted by similar reference signs, and description thereof will be omitted.

A contact lens-shaped intraocular lighting device 100Y illustrated in FIG. 8 includes a contact lens 10X including the third region 3 having a ring shape provided in contact with the sclera 73 and the second region 2 having a ring shape extending radially inward from an inner peripheral portion of the third region 3, and the irradiation unit 4B provided in the second region 2 of the contact lens 10X and irradiating an eye with light by adjusting diffusion of light supplied from the light source 52. The contact lens 10X is different from the contact lens 10 in not including the first region 1. The contact lens 10X includes, for example, the second region 2 and the third region 3, and further includes the antireflection film 30 on the surface opposite to the wearing surface 3A.

The contact lens-shaped intraocular lighting device 100Y is manufactured, for example, by a method for manufacturing the contact lens-shaped intraocular lighting device 100 and then further including a step for opening of forming an opening at the center in plan view of the contact lens 10.

The contact lens-shaped intraocular lighting device 100Y also gives similar effects to those of the contact lens-shaped intraocular lighting device 100.

Although an embodiment of the present invention has been described in detail above, the present invention is not limited to the above embodiment, and various modifications and changes can be made within the scope of the gist of the present invention described in the claims.

### INDUSTRIAL APPLICABILITY

In vitreous surgery, it has been necessary to support the intraocular lighting device with one hand in the known technique, but the use of the contact lens-shaped intraocular lighting device of the above embodiment enables bimanual surgery, which is effective.

In the scleral buckling procedure for retinal detachment, a complicated procedure using a binocular indirect ophthalmoscope in combination is required when observing the fundus in the known technique, but combined use of the contact lens-shaped intraocular lighting device of the above embodiment and a wide-angle fundus observation system enables easy fundus observation, which is effective.

### REFERENCE SIGNS LIST

1: first region, 2: second region, 3: third region, 3A: wearing surface, 4A, 4B: irradiation unit, 10, 10X: contact lens, 20: holographic lens unit, 21: holographic lens, 22: holographic lens drive circuit, 30: antireflection film, 40A, 40B: optical layered unit, 41A, 41B: light guide plate, 42A, 42B: diffusion sheet, 50: power source, 51: light source drive circuit, 52: light source, 70: cornea, 71: crystalline lens, 72: iris, 73: sclera, 100A, 100B, 100C, 100X, 100Y: contact lens-shaped intraocular lighting device

## Claims

1. A contact lens-shaped intraocular lighting device comprising:
a contact lens including a first region having a dome shape transmitting light and
provided in a center portion, a second region having a ring shape extending radially outward from an outer peripheral portion of the first region, and a third region having a ring shape extending radially outward from an outer peripheral portion of the second region and provided in contact with a sclera; and
an irradiation unit provided in the second region of the contact lens and irradiating an eye with light by adjusting refraction or diffusion of light supplied from a light source.

2. The contact lens-shaped intraocular lighting device according to claim 1, wherein the irradiation unit includes
a holographic lens refracting and guiding, into an eye, light supplied from the light source, and
a holographic lens drive circuit electrically connected with the holographic lens and changing an orientation of the holographic lens.

3. The contact lens-shaped intraocular lighting device according to claim 2, wherein
the irradiation unit includes a plurality of holographic lens units each including the holographic lens and the holographic lens drive circuit, and
a plurality of the holographic lens units are provided side by side along an identical circumference of the contact lens in plan view of the contact lens.

4. The contact lens-shaped intraocular lighting device according to claim 3, wherein a plurality of the holographic lens units are arranged rotationally symmetrically about a center of the contact lens in plan view of the contact lens.

5. The contact lens-shaped intraocular lighting device according to claim 1 or 2, further comprising a light source drive circuit provided in the second region and driving the light source.

6. The contact lens-shaped intraocular lighting device according to claim 1, wherein the irradiation unit includes
a light guide plate guiding, to a diffusion sheet, light supplied from the light source, and
a diffusion sheet layered on the light guide plate, and diffusing and guiding, into an eye, light from the light guide plate.

7. The contact lens-shaped intraocular lighting device according to claim 6, wherein
the irradiation unit includes a plurality of optical layered units each including the light guide plate and the diffusion sheet, and
a plurality of the optical layered units are provided side by side on an identical circumference of the contact lens in plan view of the contact lens.

8. The contact lens-shaped intraocular lighting device according to claim 7, wherein a plurality of the optical layered units are arranged rotationally symmetrically about a center of the contact lens in plan view of the contact lens.

9. The contact lens-shaped intraocular lighting device according to claim 1, further comprising
an antireflection film provided along an outer surface on an opposite side to a wearing surface of the contact lens, wherein
the antireflection film is arranged to cover the irradiation unit in plan view of the contact lens.

10. The contact lens-shaped intraocular lighting device according to claim 1, wherein a thickness of the first region is 0.15 mm or more and less than 0.25 mm.

11. The contact lens-shaped intraocular lighting device according to claim 1, wherein the contact lens is a scleral lens having a diameter of 13 mm or more.

12. The contact lens-shaped intraocular lighting device according to claim 1, wherein the light source is provided in the second region, and
electric power is supplied to the light source from an outside of the contact lens-shaped intraocular lighting device.

13. The contact lens-shaped intraocular lighting device according to claim 1, wherein an outer surface on an opposite side to a wearing surface of the contact lens has a spherical surface corresponding to the first region, a first ring surface corresponding to the second region, the first ring surface being a continuous surface with the spherical surface, and a second ring surface corresponding to the third region.

14. A contact lens-shaped intraocular lighting device comprising:
a contact lens including a third region having a ring shape provided in contact with a sclera, and a second region having a ring shape extending radially inward from an inner peripheral portion of the third region; and
an irradiation unit provided in the second region of the contact lens and irradiating an eye with light by adjusting refraction or diffusion of light supplied from a light source.
